# EUROPEAN PATENT SPECIFICATION

(11) **EP 1 089 711 B1**
(45) Date of publication and mention of the grant of the patent: **15.09.2004**
(21) Application number: 99926669.5
(22) Date of filing: 25.06.1999
(51) Int. Cl.: A61K 9/107, A61K 9/127, A61K 9/50

(54) **CALCIUM PHOSPHATE COATED VESICLES**
KALZIUMPHOSPHATBESCHICHTETE VESIKEL
VESICULES ENROBEES DE PHOSPHATE DE CALCIUM

(30) Priority: 26.06.1998 GB 9813906
(43) Date of publication of application: 11.04.2001
(73) Proprietor: ISIS INNOVATION LIMITED, Summertown, Oxford OX2 7BZ (GB)
(72) Inventor: CZERNUSZKA, Jan Tadeusz, Parks Road, Oxford OX1 3PH (GB); HADDOW, David Bryan, Christchurch, Dorset BH23 4AJ (GB)
(74) Representative: Ellis-Jones, Patrick George Armine
(86) International application number: PCT/GB1999/002005
(87) International publication number: WO 2000/000177

(56) References cited:
- CHEMICAL ABSTRACTS, vol. 128, no. 4, 26 January 1998 (1998-01-26) Columbus, Ohio, US; abstract no. 39477, MESSERSMITH, PHILLIP B. ET AL: "Preparation of Calcium - Loaded Liposomes and Their Use in Calcium Phosphate Formation" XP002117645 & CHEM. MATER. (1998), 10(1), 109-116 , 1998,

## Description

The present invention relates to coating materials comprising calcium phosphate, processes for their preparation and their use in coatings. The invention in particular relates to vesicles or hydrophobic droplets comprising an outer layer which comprises calcium phosphate, their preparation and their use in, for example, the coating of implants and drug delivery.

Porous monolithic ceramics based on hydroxyapatite (HA) have been shown to aid in osteoconduction of bone when implanted into a bony defect. However, they are too brittle to use in structural applications.

One way to overcome this problem is to coat a metallic implant with HA. The HA provides the bone bonding capacity while the metal provides the structural support. Unfortunately, many common methods for producing coatings require elevated temperatures which result in undesired effects such as degradation of HA to various calcium phosphate phases depending on the stoichiometry of the starting powder and the cooling rate on coating formation [R. LeGEROS, *Clinical Materials.* **14** (1993) 65].

Implant materials may be plasma sprayed with hydroxyapatite prior to being exposed to the biological environment. However, use of plasma-sprayed coatings [K. de GROOT, *J*. *Biomed Mat. Res.* **21** (1987) 1375] has shown that high processing temperatures are responsible for the drawbacks experienced with such coatings. The drawbacks include, for example, variable compositions, lack of control over the microstructure, and cracking and delamination of the coating from the substrate. The last two effects also arise due to the relative thickness of the coating resulting in poor mechanical properties across the coating thickness. In addition, plasma-sprayed coatings offer none of the porosity required to encourage bone ingrowth. They also require expensive processing equipment.

In order to utilise a wider range of substrates than the metals traditionally used, it is desirable to be able to use a coating method that does not require elevated processing temperatures. This would allow the use of materials that are traditionally considered bioinert, but which may have the necessary strength and toughness to act as implant materials. Several such methods exist, and include a biomimetic process [T. KOKUBO, in "Bone-Bonding Biomaterials" (Reed Healthcare Communications, Netherlands, 1992) 102] and electrophoretic deposition [M. SHIRKANZADEH, M. AZADEGAN, V. STACK and S. SCHREYER, *Materials Letters.* **18** (1994) 211]. However, these methods are substrate specific, and in the case of the biomimetic process the time period required for apatite growth is significant.

It is also advantageous to incorporate a degree of porosity into the coatings to encourage bone ingrowth in an implant situation. To achieve such ingrowth the porosity must be on a scale compatible with bone regeneration.

The present invention provides coating materials comprising calcium phosphate which overcome the problems discussed above. They may be used to coat substrates at low temperatures while offering a high degree of control over both the coating thickness and the degree of porosity. Such a low temperature method also allows the incorporation into the coating of other compounds which would undergo degradation at high temperatures such as, for example, heat sensitive pharmaceutically active compounds.

In a first aspect, the present invention provides a vesicle comprising
a) an inner layer which comprises a phospholipid, and
b) an outer layer which comprises calcium phosphate.

The phospholipid comprising the inner layer is any phospholipid capable of forming vesicles in an aqueous mixture, or a mixture of such phospholipids. Preferably the phospholipid is L-α-phosphatidylserine. More preferably the phospholipid is L-α-phosphatidylcholine.

The outer layer may further comprise other ions which can be incorporated to modify the properties of the calcium phosphate including anions such as carbonate, hydrogen carbonate, hydrogen phosphate, chloride and fluoride and cations such as magnesium.

The vesicles of the present invention may contain pharmaceutically active compounds including compounds which assist the binding of the coating to existing bone (bone growth factors), treat a specific bone disease or any diseased region adjacent to bone, or relieve pain. In particular, the vesicles of the present invention may contain compounds for the treatment of tumours such as ³²P or ⁸⁹Sr containing compounds, compounds for the reduction of pain arising from tumours such as narcotic analgesics (which may be administered in lower doses according to the invention as they may be administered at the site of the tumour), compounds for the reduction of osteoclast activity caused by tumour cells such as indomethacin, prostoglandins and interleuken 6 inhibitors as well as those compounds which treat specific bone diseases such as osteoporosis, for example, parathyroid hormone, vitamin D derivatives, bisphosphanates, bone morphogenetic proteins and antibiotics, or mixtures thereof.

It has been found that the vesicles of the present invention may be readily obtained by forming them in an aqueous mixture comprising a phospholipid and then calcifying said vesicles by contacting them with an aqueous solution comprising calcium and phosphate ions.

The phospholipid concentration in the aqueous mixture should be below the concentration at which agglomeration of the phospholipid may occur. Preferably the phospholipid concentration is from 5 x 10⁻⁵ to 1 x 10⁻³g per cm³, more preferably from 5 x 10⁻⁵ to 7.5 x 10⁻⁴g per cm³ and most preferably from 2.5 x 10⁻⁴ to 5 x 10⁻⁵g per cm³.

The vesicles can be formed by agitating the aqueous mixture comprising a phospholipid as described above. This can be achieved by stirring but preferably the mixture is agitated by high-frequency sound waves having a frequency and power sufficient to form an emulsion. Preferably the frequency of sonication is from 20 to 30 kHz.

The temperature of agitation should be below the boiling point of the aqueous mixture. It should also be below the temperature at which degradation of the phospholipid may occur. Preferably the temperature of agitation is below 70°C, more preferably it is below 50°C and most preferably it is about room temperature.

The agitation time is dependent upon the method of agitation and the concentration of the aqueous mixture. Preferably the mixture is agitated for a time sufficient to form an emulsion or until no further vesicles are formed i.e. a steady state is reached. If the mixture is sonicated the agitation time for a mixture with a concentration of 5 x 10⁻⁵g of phospholipid per cm³ of aqueous mixture is generally from 15 minutes to 2 hours, especially about I hour. If a mixture of the same concentration is stirred to form the vesicles then 30 minutes to 4 hours, more preferably 1 hour to 3 hours and most preferably about 2 hours.

One or more alcohols may be incorporated into the aqueous mixture to increase vesicle size in accordance with known methods. The alcohol is typically methanol, ethanol, propanol or butanol. Preferably the alcohol is ethanol. The concentration of the alcohol is generally below the concentration at which the phospholipid begins to dissolve. Preferably the alcohol concentration is no more than about 10% by volume of the aqueous mixture.

Other components which may be added to the aqueous mixture include, for example, surfactants and pharmaceutically active compounds. Preferred surfactants include anionic surfactants, for example esters of carboxyls, sulphates and phosphates. Use of surfactants can reduce the agitation time required to produce the vesicles.

Settling of the vesicles after agitation may be prevented by, for example, magnetically stirring using a low shearing rate.

Calcification of the vesicles can be carried out by contacting the phospholipid vesicles with an aqueous solution comprising calcium and phosphate (PO₄³⁻) ions. Typically the ratio of calcium to phosphate ions is from 1:1 to 2:1, preferably from 1.4:1 to 2:1 and more preferably about 1.5:1.

The source of calcium ions in the solution is any water soluble organic or inorganic calcium compound, preferably calcium chloride or calcium nitrite and more preferably calcium nitrate.

The source of phosphate ions in the solution is any water soluble phosphate compound, preferably an orthophosphate, for example, a potassium orthophosphate, especially di-potassium hydrogen orthophosphate trihydrate.

As indicated above, other ions may be incorporated into the layer comprising calcium phosphate. For example, carbonate and hydrogen phosphate ions may be added to increase the resorption rate in the body whereas chloride, fluoride and magnesium ions may be added to decrease the resorption rate.

In particular, carbonate ions may be added to the aqueous solution of calcium and phosphate ions to vary the crystallinity and stoichiometry of the calcified layer. The maximum concentration of carbonate ions will depend on pH, temperature and the presence of other ions. It will be appreciated, though, that the calcified layer is preferably a calcium phosphate layer or a substituted calcium phosphate layer. The source of carbonate ions is any soluble carbonate or hydrogen carbonate compound and is preferably potassium hydrogen carbonate or sodium hydrogen carbonate.

Calcification may be performed by simultaneously contacting the vesicles with calcium and phosphate ions or by introducing the vesicles into a calcium solution, for example, for about two hours, prior to the addition of phosphate ions.

The time for which the vesicles are contacted with the calcifying solution affects the thickness of the outer layer formed on the vesicles. Typically after about 1 hour the thickness of the layer is about 10 nm. The thickness of the layer (coupled with its porosity) may affect the rate at which the vesicles are broken down in the body and the rate of release of any pharmaceutically active compounds from within the vesicles. Preferably the coat thickness is from 5 to 50 nm, more preferably about 5 to 20 nm and most preferably about 10 nm.

In another aspect, the present invention provides a hydrophobic droplet comprising
a) a hydrophobic core,
b) an inner layer which comprises a surfactant, and
c) an outer layer which comprises calcium phosphate.

The hydrophobic core preferably comprises a solid or liquid hydrocarbon or lipid. It may further comprise water insoluble pharmaceutically active compounds.

The surfactant may be any surfactant which can reduce the surface energy of the non-aqueous droplet. It may also provide an active site for calcium phosphate deposition. Preferably the surfactant is an anionic surfactant, for example an ester of a carboxyl, sulphate or phosphate.

It has been found that the hydrophobic droplets of the present invention may be readily obtained by forming hydrophobic droplets in an aqueous mixture comprising a hydrophobic liquid or solid and a surfactant. The droplets are then calcified by contacting them with an aqueous solution comprising calcium and phosphate ions and, optionally, other ions, as discussed above.

The hydrophobic droplets of the present invention may be formed by agitation and subsequent calcification as described above.

The size of the vesicles and hydrophobic droplets of the present invention is generally from 100 nm to 10 µm, preferably at least 300 nm and more preferably at least 1 µm. Size may be increased to over 1 µm by addition of an alcohol, for example, ethanol, to the aqueous mixture prior to agitation. The size of the vesicles or droplets may also be controlled by extrusion processes using, for example, hypodermic syringes or porous membranes.

In a further, aspect the present invention provides a solid substrate wherein regions of said substrate have attached thereto a layer comprising the vesicles or droplets described above with other region or regions having no vesicles or droplets attached thereto. Such substrates may find application in the treatment of bone disorders and/or the *in vitro* delivery of pharmaceutically active compounds.

The substrates which can be coated may be electrically conductive over all or part of their surface. They may be, for example, metals such as gold, plastics or ceramics coated with metal over all or part of their surface, metals partially coated with plastic, or semi-conductors. Preferably the substrates have non-conducting regions on their surfaces of from 10 µm to 2 mm in diameter and more preferably the regions are about 150 µm in diameter.

The substrates may be coated using an electrolytic deposition process or by applying vesicles or droplets of the present invention in the form of a powder. Preferably the substrates are electrolytically coated.

The electrolytic deposition process may be carried out in an aqueous solution at a pH of from 5 to 11, preferably 6 to 8, more preferably about 7.4. The form of the calcium phosphate deposited may vary with pH. For example, at high pH hydroxyapatite may be deposited whereas at low pH brushite may be deposited.

The temperature of deposition is generally below 100°C; preferably below 70°C and more preferably about 50°C.

A salt such as, for example, potassium chloride may be added to the solution to maintain supersaturation by keeping a high background ionic strength and act as an electrolyte. Alternatively, calcium and phosphate can be added during the precipitation process to maintain supersaturation.

The coating thickness does, of course, increase with deposition time. For example, after a deposition time of 1 hour the coating thickness is about 2 µm. Multiple depositions may be performed or deposition time prolonged to access thicker coatings, for example coatings of about 20 µm.

Coatings may be formed from mixtures of the vesicles and/or droplets of the present invention. Thus different regions on the surface of the substrates may be coated with different types of vesicles or droplets. For example, a non-conducting pattern may be applied to the substrate prior to the first deposition. After the first deposition using one or more types of vesicle and/or droplet of the present invention the non-conducting pattern may be removed and a second deposition performed using different vesicles and/or droplets according to the invention. Alternatively, for example, a metal substrate is coated. It may then be subjected to a partial etching or lithographic process and a second deposition performed in a different solution of vesicles and/or droplets. Use of a variety of vesicles and/or droplets may allow the release of pharmaceutically active compounds in the coating to be controlled. For example, compounds incorporated into vesicles or droplets with a thin coating will be released more rapidly than compounds incorporated into vesicles or droplets with a thick coating.

The present invention is further illustrated, merely by way of example, with reference to the figures in which:
Figure 1 shows a schematic of the experimental apparatus used for electrolytic deposition of the vesicles and droplets of the present invention onto the surface of substrate plates.
Figure 2 shows an X-ray diffraction trace of calcified vesicles formed in a calcification solution comprising carbonate ions at atmospheric concentration. The trace shows peaks diagnostic of HA.

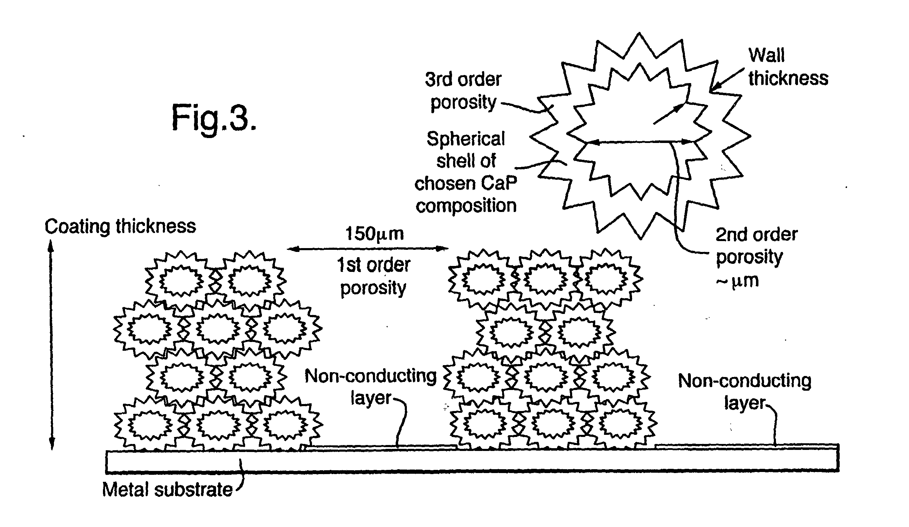

Figure 3 shows a schematic illustration of the structural hierarchy and graded porosity in the coatings of the present invention. It will be appreciated that the first order porosity concerns the spacing between the deposits. the second order porosity concerns the porosity of the vesicles before calcification and the third order porosity concerns the porosity of the calcium phosphate layer. As discussed above all of these three can be varied by appropriate manipulation of the process parameters.
The Examples which follow further illustrate the present invention with reference to the figures.

### Examples

Coatings were examined using a Jeol 840 FEG SEM using an accelerating voltage of 2 kV. Phase determination was carried out on a Phillips PW 1710 X-ray diffractometer. Fourier Transform Infra-Red spectroscopy was performed on an ATI Mattson Genesis Series Spectrometer in the wavenumber range 4000 to 400 cm⁻¹.

### Vesicle Formation

5 or 10 mg or L-α-Phosphatidycholine or L-α-Phosphatidylserine (both from Sigma, U.K.) were sonicated (i.e., agitated by high-frequency sound waves) using a Kerry Ultrasonics Ltd ultrasonic stirrer in 20 ml of distilled water for up to one hour at room temperature to form spherical vesicles. After sonication settling of the vesicles was prevented by magnetic stirring using a low shearing rate. These phospholipids are well known to show a high affinity for calcium binding.

### Calcification of Vesicles

The sonicated solution was added to a calcium phosphate (CaP) working solution. This working solution was made up as follows: solutions of 1.75 mM [Ca] were made up of Ca/P ratio=5/3 using analytical grade calcium nitrate tetrahydrate, Ca(NO₃)₂.4H₂O and di-potassium hydrogen orthophosphate trihydrate, K₂HPO₄.3H₂O (both from Aldrich, U.K.) in distilled water. Carbonate ions were incorporated using potassium hydrogen carbonate, KHCO₃ (from Adrich, U.K.). Carbonate ions were added to correspond to the following concentrations: (i) atmospheric CO₂ (1.445 x 10⁻⁷ M), and (ii) physiological [CO₃²⁻] (1.6 x 10⁻⁶ M).

### Deposition of Vesicles

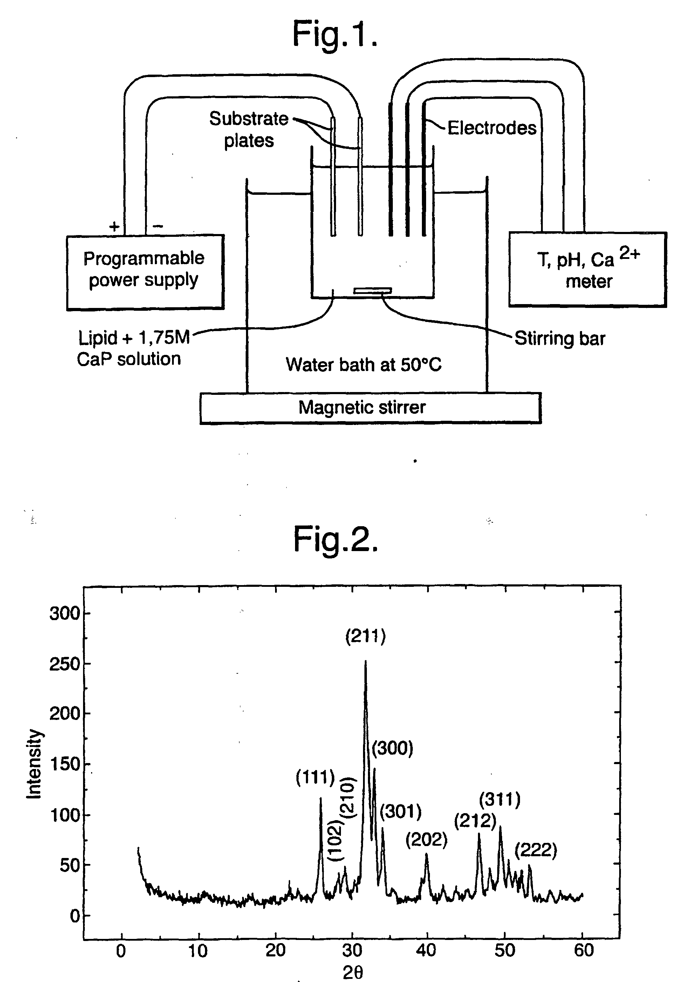

A platinum anode and a 304 stainless steel cathode were placed in the supersaturated solutions prepared as described above. The electrodes were connected to a Phillips PM2831 programmable power supply capable of both constant current (CC) and constant voltage (CV) characteristics. Calcium ion concentration, pH and temperature were monitored using an ION85 meter (Radiometer Ltd, Copenhagen), from which the supersaturation was measured. All experiments were performed at 50°C, starting pH 7.4, working solution 1.75 mM [Ca²⁺], 0.1M KCI, with a working electrode spacing of 2.25 cm and 2.2V applied potential. As well as the lipid/water solutions, additional solutions were prepared incorporating an alcohol (ethanol) into the lipid solution to be sonicated. Calcification and deposition of the vesicles was performed in the same manner as previously described. At the deposition temperature of 50°C, the alcohol slowly evaporates, resulting in an increased vesicle size. Because phospholipids are highly soluble in alcohols, the percentage volume of alcohol in the sonicated solution was limited to 10% (2 ml). The experimental set-up is detailed in Figure 1.

There was no change in the solution as the temperature was raised from the vesicle preparation temperature (ambient room temperature) up to the deposition temperature of 50°C. Because the vesicles were formed before introduction into a calcium containing media, all calcification occurred on the outer surface of the vesicles. This was confirmed by SEM examination of the coatings.

XRD and FT-IR of coatings prepared with a carbonate concentration equal to atmospheric equilibrium indicated that the deposits comprised near stoichiometric hydroxyapatite, with very low levels of carbonate. These did not show up in the XRD traces (Figure 2) but peaks attributable to CO₃²⁻ were seen in the FT-IR spectra. At higher carbonate concentrations the XRD traces were similar, but the carbonate bands in the FT-IR spectra were more intense.

SEM investigations of coatings prepared using 5 mg phospholipid indicated that surface coverage was typically less than 50%. Coatings prepared from 10 mg lipid solutions exhibited greater coverage such that the substrate was not visible under SEM. Phosphatidylcholine coatings were chosen for examination under the Jeol FEG-SEM because a comparison of five of these coatings with those prepared using phosphatidylserine showed that % cover was greater with the phosphatidylcholine.

SEM of phosphatidylcholine (10 mg) coatings showed that the vesicle size ranged from 300 nm for aqueous solutions, up to 1 µm for solutions incorporating alcohol. It is thus possible to vary the vesicle size by choice of solutions properties.

The calcified wall thickness was determined to be about 10 nm, for a vesicle suspension exposed to calcium ions for a period of one hour. The calcified wall thickness increases with exposure time to calcium ions.

Coating thickness (i.e. after impingement of spheres onto the substrate) increases with deposition time. By varying the deposition time, coatings of the required thickness may be obtained.

## Claims

1. . A vesicle comprising
a) an inner layer which comprises a phospholipid, and
b) an outer layer which comprises calcium phosphate.

2. A vesicle according to claim 1 wherein the phospholipid is selected from L-α-phosphatidylcholine and L-α-phosphatidylserine.

3. A hydrophobic droplet comprising
a) a hydrophobic core,
b) an inner layer which comprises a surfactant, and
c) an outer layer which comprises calcium phosphate.

4. A droplet according to claim 3 wherein the hydrophobic core comprises a solid or liquid hydrocarbon or lipid.

5. A droplet according to claim 3 or claim 4 wherein the surfactant is an anionic surfactant.

6. A vesicle or droplet according to any one of the preceding claims wherein the outer layer further comprises ions selected from carbonate, hydrogen phosphate, chloride, fluoride or magnesium.

7. A vesicle or droplet according to any one of the preceding claims wherein the thickness of the outer layer is from 5 to 50 nm.

8. A vesicle or droplet according to claim 7 wherein the thickness of the outer layer is from 5 to 20 nm.

9. A vesicle or droplet according to claim 8 wherein the thickness of the outer layer is about 10 nm.

10. A vesicle or droplet according to any one of the preceding claims wherein the size of the vesicle or droplet is from 100 nm to 10 µm.

11. A vesicle or droplet according to claim 10 wherein the size of the vesicle or droplet is at least 300 nm.

12. A vesicle or droplet according to claim 11 wherein the size of the vesicle or droplet is at least 1 µm.

13. A vesicle or droplet according to any one of the preceding claims which further comprises a pharmaceutically active compound.

14. A vesicle or droplet according to claim 13 wherein the pharmaceutically active compound assists the binding of a coating comprising the vesicle or droplets to bone, treats a specific bone disease or any diseased region adjacent to bone, or relieves pain.

15. A vesicle or droplet according to claim 14 wherein the pharmaceutically active compound is selected from parathyroid hormone, vitamin D derivatives, bisphosphanates, bone morphogenetic proteins, analgesics, ³²P or ⁸⁹Sr containing compounds, indomethacin, prostoglandins, interleuken 6 inhibitors and antibiotics.

16. A process for preparing a vesicle as claimed in any one of claims 1, 2 or 6 to 15, which process comprises
a) forming a vesicle in an aqueous mixture comprising a phospholipid, and
b) calcifying the vesicle by contacting said vesicle with an aqueous solution comprising calcium and phosphate ions.

17. A process for preparing a hydrophobic droplet as defined in any one of claims 3 to 15 which process comprises
a) forming a hydrophobic droplet in an aqueous mixture comprising a hydrophobic liquid or solid and a surfactant, and
b) calcifying the droplet by contacting said droplet with an aqueous solution comprising calcium and phosphate ions.

18. A process according to claim 16 or claim 17 wherein the aqueous mixture further comprises an alcohol.

19. A process according to claim 18 wherein the alcohol is selected from methanol, ethanol, propanol and butanol.

20. A process according to claim 18 or claim 19 wherein the concentration of alcohol is no more than 10% by volume of the aqueous mixture.

21. A process according to any one of claims 16 to 20 wherein the ratio of calcium to phosphate ions in the aqueous solution is from 1:1 to 2:1.

22. A process according to claim 21 wherein the ratio of calcium to phosphate ions is from 1.4:1 to 2:1.

23. A process according to claim 22 wherein the ratio of calcium to phosphate ions is about 1.5:1.

24. A vesicle according to claim 1 prepared by the process according to any one of claims 16 or 18 to 23.

25. A droplet according to claim 3 prepared by the process according to any one of claims 17 to 23.

26. A solid substrate wherein regions of said substrate have attached thereto a layer comprising vesicles or droplets as claimed in any one of claims 1 to 15, 24 or 25 with other region or regions having no vesicles or droplets attached thereto.

27. A substrate according to claim 26 which comprises
a) electrically conducting and non-conducting regions on its surface, and
b) a layer comprising vesicles or droplets on the conducting regions.

28. A substrate according to claim 27 wherein the non-conducting regions are from 10 µm to 2 mm in size.

29. A substrate according to claim 28 wherein the non-conducting regions are about 150 µm in size.

30. A process for preparing a substrate according to any one of claims 26 to 29 which process comprises electrolytically depositing the coating comprising vesicles or droplets onto the conducting regions of the substrate.

31. A substrate according to claim 26 prepared by the process according to claim 30.

32. A substrate according to any one of claims 26 to 29 for use in the treatment of the human or animal body.

33. Use of a substrate according to any one of claims 26 to 29 in the manufacture of a medically acceptable implant the treatment of bone disorders or in the delivery of pharmaceutically active compounds.

## Patentansprüche

1. Bläschen, welches umfasst:
(a) eine Innenschicht, welche ein Phospholipid umfasst, und
(b) eine Außenschicht, welche Calciumphosphat umfasst.

2. Bläschen nach Anspruch 1, wobei das Phospholipid ausgewählt ist aus L-α-Phosphatidylcholin und L-α-Phosphatidylserin.

3. Hydrophobes Tröpfchen, welches umfasst:
(a) einen hydrophoben Kern,
(b) eine lnnenschicht, welche ein oberflächenaktives Mittel umfasst, und
(c) eine Außenschicht, welche Calciumphosphat umfasst.

4. Tröpfchen nach Anspruch 3, wobei der hydrophobe Kern einen festen oder flüssigen Kohlenwasserstoff oder Lipid umfasst.

5. Tröpfchen nach Anspruch 3 oder Anspruch 4, wobei das oberflächenaktive Mittel ein anionisches oberflächenaktives Mittel ist.

6. Bläschen oder Tröpfchen nach einem der vorangehenden Ansprüche, wobei die Außenschicht außerdem lonen umfasst, ausgewählt aus Carbonat, Hydrogenphosphat, Chlorid, Fluorid oder Magnesium.

7. Bläschen oder Tröpfchen nach einem der vorangehenden Ansprüche, wobei die Dicke der Außenschicht 5 bis 50 nm beträgt.

8. Bläschen oder Tröpfchen nach Anspruch 7, wobei die Dicke der Außenschicht 5 bis 20 nm beträgt.

9. Bläschen oder Tröpfchen nach Anspruch 8, wobei die Dicke der Außenschicht etwa 10 nm beträgt.

10. Bläschen oder Tröpfchen nach einem der vorangehenden Ansprüche, wobei die Größe des Bläschens oder Tröpfchens 100 nm bis 10 µm beträgt.

11. Bläschen oder Tröpfchen nach Anspruch 10, wobei die Größe des Bläschens oder Tröpfchens mindestens 300 nm beträgt.

12. Bläschen oder Tröpfchen nach Anspruch 11, wobei die Größe des Bläschens oder Tröpfchens mindestens 1 µm beträgt.

13. Bläschen oder Tröpfchen nach einem der vorangehenden Ansprüche, welches außerdem eine pharmazeutisch wirksame Verbindung umfasst.

14. Bläschen oder Tröpfchen nach Anspruch 13, wobei die pharmazeutisch wirksame Verbindung das Binden einer Beschichtung, umfassend die Bläschen oder Tröpfchen, an Knochen unterstützt, eine spezifische Knochenerkrankung oder eine an den Knochen angrenzende erkrankte Region behandelt, oder Schmerz lindert.

15. Bläschen oder Tröpfchen nach Anspruch 14, wobei die pharmazeutisch wirksame Verbindung ausgewählt ist aus Parathormon, Vitamin-D-Derivaten, Biphosphanaten, morphogenetischen Knochenproteinen, Analgetika, ³²P- oder ⁸⁹Sr-haltigen Verbindungen, Indomethacin, Prostaglandinen, Interleukin-6-Inhibitoren und Antibiotika.

16. Verfahren zum Herstellen eines Bläschens nach einem der Ansprüche 1, 2 oder 6 bis 15, welches Verfahren umfasst:
(a) Bilden eines Bläschens in einem wässrigen Gemisch, welches ein Phospholipid umfasst, und
(b) Calcifizieren des Bläschens durch Zusammenbringen des Bläschens mit einer wässrigen Lösung, welche Calcium- und Phosphationen umfasst.

17. Verfahren zum Herstellen eines hydrophoben Tröpfchens nach einem der Ansprüche 3 bis 15, welches Verfahren umfasst:
(a) Bilden eines hydrophoben Tröpfchens in einem wässrigen Gemisch, welches eine hydrophobe Flüssigkeit oder Feststoff und ein oberflächenaktives Mittel umfasst, und
(b) Calcifizieren des Tröpfchens durch Zusammenbringen des Tröpfchens mit einer wässrigen Lösung, welche Calcium- und Phosphationen umfasst.

18. Verfahren nach Anspruch 16 oder Anspruch 17, wobei das wässrige Gemisch außerdem einen Alkohol umfasst.

19. Verfahren nach Anspruch 18, wobei der Alkohol ausgewählt ist aus Methanol, Ethanol, Propanol und Butanol.

20. Verfahren nach Anspruch 18 oder Anspruch 19, wobei die Konzentration des Alkohols nicht mehr als 10 Volumen-% des wässrigen Gemischs beträgt.

21. Verfahren nach einem der Ansprüche 16 bis 20, wobei das Verhältnis von Calcium- zu Phosphationen in der wässrigen Lösung von 1:1 bis 2:1 beträgt.

22. Verfahren nach Anspruch 21, wobei das Verhältnis von Calcium- zu Phosphationen von 1,4:1 bis 2:1 beträgt.

23. Verfahren nach Anspruch 22, wobei das Verhältnis von Calcium- zu Phosphationen etwa 1,5:1 beträgt.

24. Bläschen nach Anspruch 1, hergestellt mittels des Verfahrens nach einem der Ansprüche 16 oder 18 bis 23.

25. Tröpfchen nach Anspruch 3, hergestellt mittels des Verfahrens nach einem der Ansprüche 17 bis 23.

26. Festes Substrat, wobei Regionen des Substrats eine daran gebundene Schicht aufweisen, welche Bläschen oder Tröpfchen nach einem der Ansprüche 1 bis 15, 24 oder 25 umfasst, wobei eine andere Region oder Regionen keine daran gebundenen Bläschen oder Tröpfchen aufweisen.

27. Substrat nach Anspruch 26, welches umfasst
(a) elektrisch leitende und nicht-leitende Regionen auf seiner Oberfläche, und
(b) eine Schicht, welche Bläschen oder Tröpfchen auf den leitenden Regionen umfasst.

28. Substrat nach Anspruch 27, wobei die nicht-leitenden Regionen von 10 µm bis 2 mm groß sind.

29. Substrat nach Anspruch 28, wobei die nicht-leitenden Regionen etwa 150 µm groß sind.

30. Verfahren zum Herstellen eines Substrats nach einem der Ansprüche 26 bis 29, welches Verfahren das elektrolytische Niederschlagen der Beschichtung, welche Bläschen oder Tröpfchen umfasst, auf die leitenden Regionen des Substrats umfasst.

31. Substrat nach Anspruch 26, hergestellt mittels des Verfahrens nach Anspruch 30.

32. Substrat nach einem der Ansprüche 26 bis 29 zur Verwendung in der Behandlung des menschlichen oder tierischen Körpers.

33. Verwendung eines Substrats nach einem der Ansprüche 26 bis 29 in der Herstellung eines medizinisch geeigneten Implantats für die Behandlung von Knochenschädigungen oder bei der Darreichung von pharmazeutisch wirksamen Verbindungen.

## Revendications

1. Vésicule comprenant
a) une couche interne qui comprend un phospholipide, et
b) une couche externe qui comprend du phosphate de calcium.

2. Vésicule selon la revendication 1 dans laquelle le phospholipide est choisi parmi la L-α-phosphatidylcholine et la L-α-phosphatidylsérine.

3. Gouttelette hydrophobe comprenant
a) un coeur hydrophobe,
b) une couche interne qui comprend un agent tensio-actif, et
c) une couche externe qui comprend du phosphate de calcium.

4. Gouttelette selon la revendication 3 dans laquelle le coeur hydrophobe comprend un hydrocarbure ou un lipide solide ou liquide.

5. Gouttelette selon la revendication 3 ou la revendication 4 dans laquelle l'agent tensio-actif est un agent tensio-actif anionique.

6. Vésicule ou gouttelette selon l'une quelconque des revendications précédentes dans laquelle la couche externe comprend en outre des ions choisis parmi le carbonate, l'hydrogénophosphate, le chlorure, le fluorure ou le magnésium.

7. Vésicule ou gouttelette selon l'une quelconque des revendications précédentes dans laquelle l'épaisseur de la couche externe est entre 5 et 50 nm.

8. Vésicule ou gouttelette selon la revendication 7 dans laquelle l'épaisseur de la couche externe est entre 5 et 20 nm.

9. Vésicule ou gouttelette selon la revendication 8 dans laquelle l'épaisseur de la couche externe est d'environ 10 nm.

10. Vésicule ou gouttelette selon l'une quelconque des revendications précédentes dans laquelle la taille de la vésicule ou gouttelette est entre 100 nm et 10 µm.

11. Vésicule ou gouttelette selon la revendication 10 dans laquelle la taille de la vésicule ou gouttelette est d'au moins 300 nm.

12. Vésicule ou gouttelette selon la revendication 11 dans laquelle la taille de la vésicule ou gouttelette est d'au moins 1 µm.

13. Vésicule ou gouttelette selon l'une quelconque des revendications précédentes qui comprend en outre un composé pharmaceutiquement actif.

14. Vésicule ou gouttelette selon la revendication 13 dans laquelle le composé pharmaceutiquement actif aide à la liaison d'un revêtement comprenant la vésicule ou les gouttelettes à un os, traite une maladie spécifique de l'os ou une région malade quelconque adjacente à l'os, ou soulage la douleur.

15. Vésicule ou gouttelette selon la revendication 14 dans laquelle le composé pharmaceutiquement actif est choisi parmi la parathormone, les dérivés de la vitamine D, les bisphosphanates, les protéines morphogénétiques d'os, les analgésiques, les composés contenant ³²P ou ⁸⁹Sr, l'indométhacine, les prostoglandines, les inhibiteurs d'interleukine 6 et les antibiotiques.

16. Procédé de préparation d'une vésicule selon l'une quelconque des revendications 1, 2 ou 6 à 15, lequel procédé comprend
a) la formation d'une vésicule dans un mélange aqueux comprenant un phospholipide, et
b) la calcification de la vésicule par mise en contact de la dite vésicule avec une solution aqueuse comprenant des ions calcium et phosphate.

17. Procédé de préparation d'une gouttelette hydrophobe selon l'une quelconque des revendications 3 à 15, lequel procédé comprend
a) la formation d'une gouttelette hydrophobe dans un mélange aqueux comprenant un liquide ou solide hydrophobe et un agent tensio-actif, et
b) la calcification de la gouttelette par mise en contact de ladite gouttelette avec une solution aqueuse comprenant des ions calcium et phosphate.

18. Procédé selon la revendication 16 ou la revendication 17 dans lequel le mélange aqueux comprend en outre un alcool.

19. Procédé selon la revendication 18 dans lequel l'alcool est choisi parmi le méthanol, éthanol, propanol et butanol.

20. Procédé selon la revendication 18 ou la revendication 19 dans lequel la concentration d'alcool n'est pas supérieure à 10 % en volume du mélange aqueux.

21. Procédé selon l'une quelconque des revendications 16 à 20 dans lequel le rapport des ions calcium par rapport au phosphate dans la solution aqueuse est de 1 : 1 à 2: 1.

22. Procédé selon la revendication 21 dans lequel le rapport des ions calcium par rapport au phosphate est de 1,4 : 1 à 2 : 1.

23. Procédé selon la revendication 22 dans lequel le rapport des ions calcium par rapport au phosphate est d'environ 1,5 : 1.

24. Vésicule selon la revendication 1 préparée par le procédé selon l'une quelconque des revendications 16 ou 18 à 23.

25. Gouttelette selon la revendication 3 préparée par le procédé selon l'une quelconque des revendications 17 à 23.

26. Substrat solide dans lequel est déposée sur des régions dudit substrat une couche comprenant des vésicules ou des gouttelettes selon l'une quelconque des revendications 1 à 15, 24 ou 25 avec une autre région ou d'autres régions ne portant pas de vésicules ni de gouttelettes.

27. Substrat selon la revendication 26 qui comprend
a) des régions électriquement conductrices et non conductrices sur sa surface, et
b) une couche comprenant des vésicules ou des gouttelettes sur les régions conductrices.

28. Substrat selon la revendication 27 dans lequel les régions non conductrices ont une taille allant de 10 µm à 2 mm.

29. Substrat selon la revendication 28 dans lequel les régions non conductrices ont une taille d'environ 150 µm.

30. Procédé de préparation d'un substrat selon l'une quelconque des revendications 26 à 29, lequel procédé comprend le dépôt électrolytique du revêtement comprenant des vésicules ou des gouttelettes sur les régions conductrices du substrat.

31. Substrat selon la revendication 26 préparé par le procédé selon la revendication 30.

32. Substrat selon l'une quelconque des revendications 26 à 29 pour son utilisation dans le traitement du corps humain ou animal.

33. Utilisation d'un substrat selon l'une quelconque des revendications 26 à 29 pour la fabrication d'un implant médicalement acceptable dans le traitement des troubles osseux ou dans l'administration de composés pharmaceutiquement actifs.
